# EUROPÄISCHE PATENTSCHRIFT

(11) **EP 2 552 195 B1**
(45) Veröffentlichungstag und Bekanntmachung des Hinweises auf die Patenterteilung: **06.01.2016**
(21) Anmeldenummer: 11710784.7
(22) Anmeldetag: 25.03.2011
(51) Int. Cl.: A01N 25/04, A61K 9/107, A61K 9/70, A61K 47/24, A61K 47/44, A01N 47/44

(54) **ANTIMIKROBIELLE ÖL IN WASSER EMULSION**
ANTIMICROBIAL OIL-IN-WATER EMULSION
ÉMULSION HUILE DANS L'EAU ANTIMICROBIENNE

(30) Priorität: 26.03.2010 DE 102010013081
(43) Veröffentlichungstag der Anmeldung: 06.02.2013
(73) Patentinhaber: B. Braun Melsungen AG, 34212 Melsungen (DE)
(72) Erfinder: SCHMITT, Jürgen, 35274 Kirchhain (DE); HENRICH, Yang, 34266 Niestetal (DE); ARNDT, Andreas, 6003 Luzern (CH); BRILL, Florian, H.H., 6017 Ruswil (CH); KRAMER, Axel, 17489 Greifswald (DE); MÜLLER, Gerald, 17498 Hinrichshagen (DE); LADEMANN, Olaf, 18057 Rostock (DE); SIPPEL, Martin, 34212 Melsungen (DE); RIEMANN, Thomas, 37247 Großalmerode (DE); WEIß, André, 34302 Guxhagen (DE)
(74) Vertreter: von Kreisler Selting Werner - Partnerschaft von Patentanwälten und Rechtsanwälten mbB
(86) Internationale Anmeldenummer: PCT/EP2011/054625
(87) Internationale Veröffentlichungsnummer: WO 2011/117389

(56) Entgegenhaltungen:
- WO-A1-2007/115635
- WO-A1-2008/119401
- WO-A2-02/055055

## Beschreibung

Die Erfindung betrifft eine antimikrobielle Öl in Wasser Emulsion, eine pharmazeutische Zubereitung sowie ein Antiseptikum. Darüber hinaus betrifft die Erfindung die Verwendung der antimikrobiellen Öl in Wasser Emulsion in Produkten zur Wundbehandlung .

Polyhexamethylenbiguanidiniumhydrochlorid (nachfolgend Polihexanid oder auch PHMB genannt) gilt als Lokalantiseptikum, das ein breites Wirkungsspektrum und eine gute Verträglichkeit aufweist. Applikationsfertige PHMB-Lösungen zur Wundbehandlung sind im Handel unter dem Namen Lavasept^{®} (B. Braun Melsungen AG, Melsungen, DE), Lavanid^{®} (Serag-Wiessner KG, Naila, DE) und Prontosan^{®} (B. Braun Melsungen AG, Melsungen, DE) erhältlich. Der in den Fertigpräparaten enthaltene Wirkstoff PHMB ist als 20%ige wässrige Lösung als Cosmocil^{®} in verschiedenen Qualitätsstufen von ARCH Chemicals, Inc. (USA) zu beziehen.

Die präoperative Hautantiseptik wird nach dem Stand der Technik mit rein alkoholischen Lösungen, mit alkoholischen Lösungen mit Zusatz remanenter Wirkstoffe wie Octenidindihydrochlorid oder auch mit wässrigen Povidon-Iod-Lösungen durchgeführt. Untersuchungen der Bakterienverteilung in der menschlichen Haut haben gezeigt, dass eine Vielzahl von Bakterien in den Haarfollikeln angesiedelt sind. Gerade hier erwies sich die präoperative Hautantiseptik nach Stand der Technik als nicht ausreichend, da die eingesetzten Hautantiseptika nur geringfügig in die Haarfollikel eindringen und es dadurch zu einer raschen Rekolonisation der Haut im Operations-Areal kommt.

PHMB ist ein polymerer kationischer antimikrobieller Wirkstoff, der an der Oberfläche der Zytoplasma-Membran von Mikroorganismen bindet und benachbarte Phospholipid-Kopfgruppen über eine Brücke verbindet, wobei die Bindung vorzugsweise an saure/anionische Phospholipide erfolgt. Dadurch wird die Permeabilität der Zellmembran erhöht und über den damit verbundenen erhöhten Verlust von Kalium- und Wasserstoff-Ionen sowie Zytoplasma-Bestandteilen der Zelltod von Mikroorganismen hervorgerufen.

Nachteilig bei der Verwendung von PHMB-Lösungen zur Schleimhaut- und Wundantiseptik ist jedoch, dass neben der erwünschten mikrobioziden Wirkung eine vergleichbare Wechselwirkung mit den Phospholipiden der Zellen von Menschen und Tieren stattfindet, die dann eine unerwünschte zytotoxische Reaktion hervorruft.

Eine Möglichkeit, unerwünschte zytotoxische Wirkungen zu minimieren, besteht allgemein darin, den antimikrobiellen Wirkstoff in Liposomen zu verkapseln.

Eine Möglichkeit, diesen unerwünschten Effekt zu mindern, besteht außerdem darin, dass die Zusammensetzung der liposomalen Membran so gewählt wird, dass eine zielgerichtete Wechselwirkung mit der Zytoplasmamembran oder Bestandteilen von Mikroorganismen erreicht wird, damit der liposomal verkapselte Wirkstoff direkt auf die Mikroorganismen übertragen wird.

DE 10 2005 045 146 A1 und DE 10 2005 063 375 A1 beschreiben Zubereitungen, bei denen Octenidindihydrochlorid in Liposomen verkapselt vorliegt. Da Liposomen in der Regel Phospholipide und Lipide enthalten, mit denen Biguanide wie PHMB in Wechselwirkung treten, kann davon ausgegangen werden, dass mit PHMB beladene Liposomen nicht mehr antimikrobiell wirksam sind. So ist in der DE 10 2006 015 271 A1 ein Hinweis gegeben, das die Liposomen beim Beladen mit PHMB destabilisiert werden und darüber hinaus eine rasche Inaktivierung von PHMB in Gegenwart von Phospholipiden mit anionischen Kopfgruppen und anderen anionischen Substanzen erfolgt.

DE 10 2006 015 271 versucht dieses Problem dadurch zu lösen, dass die antiseptische Zubereitungen auf der Basis eines in Liposomen eingeschlossenen (verkapselten) Wirkstoffes beschrieben wird, wobei die Liposomen keine Phospholipide mit anionischen Kopfgruppen enthalten, in ihrem Inneren ein wässriges Milieu aufweisen und in dem wässrigen Milieu mindestens ein antimikrobieller Wirkstoff aus der Gruppe der Biguanide enthalten ist. Neben der aufwändigen mehrstufigen Herstellung der Liposomen bei gleichzeitiger Anwesenheit der Wirkstoffe, zeigen diese Formulierungen ein weiteres Problem, nämlich dass die Liposomen mit dem verkapselten Wirkstoff nach ihrer Applikation zerfallen, und dass in den Liposomen verkapselte PHMB freigesetzt wird und dann in analoger Weise, wie das nicht in Liposomen eingekapselte PHMB wirkt.

DE 10 2007 030 931 beschreibt den Einsatz von Phospholipiden als nutritiven Wirkstoff in Zubereitungen für die Wundbehandlung, die gleichzeitig antimikrobielle Wirksubstanzen enthalten. Allerdings liegen diese Formulierungen nicht als Öl in Wasser Emulsion vor und es wird auch kein Hinweis gegeben, wie die immanente Zytotoxizität der antimikrobiellen Wirkstoffe gesenkt werden kann.

WO02/055055 beschreibt kosmetische Formulierungen, die als Öl in Wasser Emulsionen vorliegen können. Als aromatisches antibakterielles Mittel wird in den Beispielen Chlorhexidin offenbart.

Aromatische Biguanide, wie beispielsweise Chlorhexidin, unterscheiden sich von aliphatischen Biguaniden, wie beispielsweise PHMB, durch ihre Wirkmechanismen, der unter anderem durch unterschiedliche Wechselwirkungen mit den Phospholipiden der Plasmamembran von Mikroorganismen bedingt wird (Gilbert P & Moore LE., Cationic antiseptics: diversity of action under a common epithet. J Appl Microbiol. 99(2005) 703-715). Die Folge ist, dass sich die Wirkstoffe grundsätzlich unterschiedlich verhalten.

Es wurde nun überraschend gefunden, dass Phospholipide, die Öltröpfchen emulgieren und dabei eine Monolayer (Liposomen bilden Bilayer aus) ausbilden, sowohl Chlorhexidin als auch PHMB binden. Eine solche Bindung wurde bisher ausgeschlossen (Ikeda T, Ledwith A, Bamford CH, Hann RA, "Interaction of polymeric biguanide biocide with phopholipid membranes. I. Fluorescence depolarization studies on the effect of polymeric biocide bearing groups in the main chain. Biochim Biophys Acta 769(1984) 57-66). Im Unterschied zu Chlorhexidin bleibt das gebundene PHMB antimikrobiell wirksam und Chlorhexidin wird durch diese Bindung inaktiviert.

Aufgabe der vorliegenden Erfindung war es, Zubereitungen auf der Basis von Guaniden und Biguaniden, zur Verfügung zu stellen, die unter Beibehaltung der mikrobioziden Wirksamkeit eine deutlich verbesserte Verträglichkeit, d.h. eine geringe bzw. keine Zytotoxizität, besitzen. Darüber hinaus war es Aufgabe der vorliegenden Erfindung, eine antimikrobielle Zubereitung zur Verfügung zu stellen, die in der Lage ist, den antimikrobiellen Wirkstoff in die Haarfollikel der humanen Haut zu transportieren, um dort seine Wirkung zu entfalten.

Überraschend wurden die vorstehend genannten Aufgaben durch eine antimikrobielle Öl in Wasser Emulsion gelöst, die
a) 0,005 bis 0,2 Gew.-% mindestens einer aliphatischen Komponente, die eine oder mehrere Guanid- und/oder Biguanid-Gruppe(n) aufweist,
b) mindestens ein Phospholipid und
c) mindestens eine Ölkomponente aufweist, wobei das Gewichtsverhältnis von Komponente a) zu Komponente b) 1:4 bis 1:40 ist.

Die erfindungsgemäße Öl in Wasser Emulsion besitzt eine hervorragende mikrobizide Wirkung, zeigt aber gleichzeitig nur eine sehr geringe Zytotoxizität. Die erfindungsgemäßen antimikrobiellen Öl in Wasser Emulsionen sind daher zur Therapie und Prophylaxe von Infektionen im human- und veterinärmedizinischen Bereich und insbesondere für die Applikation an bekanntermaßen empfindlichen Körperregionen, z.B. zur Prophylaxe und Therapie von Peritonitis und/oder der Spülung anderer Körperhöhlen, einschließlich der Harnblase sowie der Mund- und Nasenhöhlen, geeignet.

Die Öl in Wasser Emulsionen dienen als Trägersubstanzen, die den antimikrobiellen Wirkstoff, also das Guanid- und/oder die Biguanid-Komponente, gebunden halten und dadurch die Wechselwirkung des antimikrobiellen Wirkstoffs mit Säuger- und humanen Zellen neutralisieren.

Die Nanopartikel der Öl in Wasser Emulsion simulieren überraschenderweise als "Geisterzellen" Säuger- und menschliche Zellen, die dadurch zum Target für die Guanid- und/oder Biguanid-Komponenten werden und verhindern damit die Wechselwirkung des antimikrobiellen Wirkstoffs mit Säuger- und humanen Zellen. Dadurch wird die bevorzugte Wechselwirkung der Guanid- und/oder Biguanid-Komponente mit den Phospholipiden der Zytoplasmamembran der Mikroorganismen erzielt. Überraschenderweise kommt es im Ergebnis zu einer selektiven mikrobioziden Wirkung.

Ein wesentlicher Bestandteil der antimikrobiellen Öl in Wasser Emulsion gemäß der vorliegenden Erfindung ist eine Ölkomponente (Komponente c)).

### Komponente c)

Prinzipiell eignen sich für die antimikrobiellen Öl in Wasser Emulsionen jegliche Ölkomponenten, bevorzugt sind jedoch Ölkomponenten natürlichen Ursprungs. Besonders bevorzugte Ölkomponenten sind Triglyceride, die Fettsäurereste tragen. Es hat sich als besonders vorteilhaft herausgestellt, insbesondere bezüglich der physiologischen Verträglichkeit, wenn die Ölkomponente ein mittelkettiges Triglycerid (MCT) umfasst. Bevorzugt weist das mittelkettige Triglycerid eine Struktur auf, die ein Glycerin ist, welches mit Fettsäuren verestert ist, die 6 bis 14 Kohlenstoffatome aufweisen. Besonders bevorzugt ist das mittelkettige Triglycerid, ein Glycerin, welches mit Fettsäuren verestert ist, die zumindest 90 mol.-% aus Caprylsäure (C₈) und Caprinsäure (C₁₀) bestehen.

Weiterhin bevorzugt sind als Ölkomponente Pflanzenöle einzusetzen. Besonders geeignet für die antimikrobiellen Öl in Wasser Emulsionen der vorliegenden Erfindung sind die Pflanzenöle ausgewählt aus der Gruppe bestehend aus Sojabohnenöl und Saffloröl oder Mischungen hiervon.

Es hat sich auch gezeigt, dass auch Mischungen von verschiedenen Ölen Vorteile zeigen. So ist etwa die Mischung aus mittelkettigem Triglycerid und zusätzlichem Pflanzenöl, insbesondere Sojabohnenöl und/oder Saffloröl, besonders physiologisch verträglich und zeigt ein optimales Wirkungsspektrum. In einer bevorzugten Ausführungsform liegt das Gewichtsverhältnis von mittelkettigem Triglycerid zu Pflanzenöl bei 1:10 bis 10:1, vorzugsweise 5:1 bis 1:5, weiter bevorzugt 2:1 bis 1:2, besonders bevorzugt 1,5:1 bis 1:1,5 und im Speziellen bei 1:1.

Die antimikrobielle Öl in Wasser Emulsion umfasst die Ölkomponente üblicherweise in einer Menge von 1 bis 30 Gew.-%, vorzugsweise 2 bis 20 Gew.-%, insbesondere 4 bis 15 Gew.-% und im Speziellen von 5 bis 10 Gew.-%, jeweils bezogen auf das Gesamtgewicht der Emulsion.

Als weitere wesentliche Komponente umfassen die Öl in Wasser Emulsionen mindestens ein Phospholipid (Komponente b)).

### Komponente b)

Phospholipide sind phosphorhaltige, amphiphile Lipide. Sie sind im Organismus als Membranlipide am Aufbau der Doppellipidschicht einer Biomembran beteiligt. Sie setzen sich aus einem hydrophilen Kopf und zwei hydrophoben Kohlenwasserstoffschwänzen zusammen.

Erfindungsgemäß ist die Komponente b) ein Phospholipid, das üblicherweise ausgewählt ist aus der Gruppe bestehend aus Phosphatidylcholin, Phosphatidylethanolamin, Phosphatidylserin, Phosphatidylinositol und Sphingomyelin sowie beliebige Mischungen hiervon.

Besonders bevorzugt ist die Komponente b), ein Lecithin, das insbesondere von Sojabohnen und/oder Eiern stammt.

Lecithin ist der klassische Name für eine Gruppe chemischer Verbindungen, die sogenannten Phosphatidylcholine. Dabei handelt es sich um Lipide, genauer Phospholipide, die aus Fettsäuren, Glycerin, Phosphorsäure und Cholin zusammengesetzt sind. Lecithine sind Bestandteile der Zellmembranen tierischer und pflanzlicher Lebewesen.

In einer besonders bevorzugten Ausführungsform weist die erfindungsgemäß einzusetzende Komponente b) Lecithin auf, welches mit natürlichen Fettsäuren verestert ist.

Erfindungsgemäß enthalten die Öl in Wasser Emulsionen die Komponente b) üblicherweise in einer Menge von 0,1 bis 5 Gew.-%, vorzugsweise 0,2 bis 2,5 Gew.-%, weiter bevorzugt 0,3 bis 1,5 Gew.-% und insbesondere 0,4 bis 1,2 Gew.-%, jeweils bezogen auf das Gesamtgewicht der Emulsion.

Ein weiterer wesentlicher Bestandteil der erfindungsgemäßen antimikrobiellen Öl in Wasser Emulsion ist die Komponente, die eine oder mehrere Guanid- oder Biguanid-Gruppe(n) aufweist (Komponente a)). Bei der Komponente a) handelt es sich um eine aliphatische Komponente, dass heißt die Komponenten weisen kein aromatisches Ringsystem auf. "Aliphatisch" im Rahmen der vorliegenden Erfindung umfasst auch cycloaliphatische oder auch heterocyclische Systeme, sofern diese keine aromatischen Ringsysteme aufweisen.

### Komponente a)

Im Rahmen der vorliegenden Erfindung handelt es sich um eine Komponente, die eine Guanid-Gruppe aufweist, wenn die Komponente in ihrer chemischen Struktur das folgende Strukturelement aufweist:

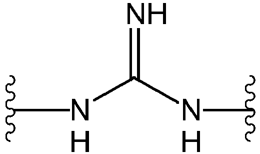

Nachfolgend werden diese Komponenten vereinfachend als "Guanid" oder "Guanide" bezeichnet.

Unter Komponenten, die eine Biguanidgruppe aufweisen sind solche chemische Verbindungen zu verstehen, die das folgende Strukturelement aufweisen:

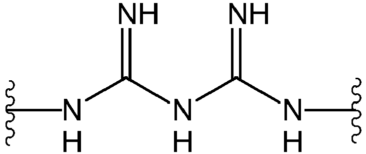

Nachfolgend werden diese Komponenten vereinfachend als "Biguanid" oder "Biguanide" bezeichnet.

Guanide und Biguanide sind als antimikrobielle Wirkstoffe im Stand der Technik bekannt. Die Guanide und Biguanide können als Monomere oder bevorzugt als Polymere, also als Polyguanid und/oder Polybiguanid eingesetzt werden. Die Guanide und/oder Biguanide liegen in einer bevorzugten Ausführungsform als wasserlösliche physiologische akzeptable Salze vor. Besonders bevorzugt liegen die Guanide und Biguanide als Hydrohalogenide, beispielsweise Hydrochlorid oder Hydrobromid vor. Bevorzugt sind auch die Hydroxide, die beispielsweise erhältlich sind durch basischen Anionenaustausch aus den entsprechenden Hydrohalogeniden.

In einer besonders bevorzugten Ausführungsform ist das erfindungsgemäß einzusetzende Biguanid ein polymeres Biguanid, welches besonders bevorzugt ein mittleres Molekulargewicht von 500 bis 20000 g/mol, weiter bevorzugt 1000 bis 15000 g/mol und insbesondere 1600 bis 10000 g/mol aufweist.

Im Rahmen der vorliegenden Erfindung speziell bevorzugt ist als Komponente a) das Polyhexamethylenbiguanid und insbesondere das Hydrochlorid, nämlich Polyhexamethylenbiguanidiumhydrochlorid (PHMB). Es können aber auch Mischungen aus Polyhexamethylenbiguanid und Polyhexamethylenbiguanidiniumhydrochlorid bevorzugt eingesetzt werden.

Überraschend hat sich auch gezeigt, dass Komponenten a) (antimikrobielle Wirkstoffe), die erhältlich sind durch Polykondensation eines Guanidinsäureadditionssalzes mit einem Amingemisch, welches wenigstens ein Diamin und/oder ein Triamin enthält, wobei vorzugsweise wenigstens ein Amin ausgewählt ist aus der Gruppe, die aus
i) Diamin, das wenigstens einen cycloaliphatischen Rest aufweist, und
ii) Dialkylentriamin besteht,
besonders geeignet sind.

Die Polykondensation kann dazu beispielsweise bei Temperaturen von 100°C bis 180°C, vorzugsweise 130°C bis 160°C über einen Zeitraum von bevorzugt 30 Minuten bis 6 Stunden durchgeführt werden.

Die so erhältlichen polymeren oder oligomeren antimikrobiellen Wirkstoffe können sowohl als Homopolymer als auch als Copolymer vorliegen. Vorteilhaft ist dabei, wenn das Guanidin-Säureadditionssalz Guanidiniumhydrochlorid ist. Es sind aber auch weitere Guanidin-Säureadditionssalze, auf Basis anorganischer oder organischer Säuren ebenfalls geeignet. Beispielsweise die Hydroxide, Hydrogensulfate sowie Acetate.

Diese antimikrobiellen Wirkstoffe zeichnen sich durch eine besonders hohe Biozidie aus und sind daher für die erfindungsgemäßen Öl in Wasser Emulsionen besonders geeignet.

Bevorzugt sind die antimikrobiellen Wirkstoffe erhältlich durch Polykondensation eines Guanidin-Säureadditionssalzes mit einem Amingemisch, welches wenigstens ein Diamin und/oder ein Triamin enthält, wobei wenigstens ein Amin ausgewählt ist aus der Gruppe, die aus
i) Diamin, das wenigstens einen cycloaliphatischen Rest aufweist, und
ii) Dialkylentriamin besteht.

Die durch die Polykondensation erhältlichen polymeren oder oligomeren Wirkstoffe weisen hierbei bevorzugt eine Polyguanidinstruktur oder, insbesondere im Falle des Einsatzes von Dialkylentriaminen, beispielsweise Diethylentriamin, eine Poly(iminoimidazolidin)-Struktur auf.

In einer bevorzugten Ausgestaltung der vorliegenden Erfindung umfasst das Amingemisch die Komponente i) (Diamin, das wenigstens einen cycloaliphatischen Rest aufweist) und/oder die Komponente ii) (Dialkylentriamin) in einer Menge von wenigstens 10 mol-%, bevorzugt mindestens 25 mol-%, weiter bevorzugt mindestens 45 mol-%, insbesondere mindestens 85 mol-%, im Speziellen mindestens 95 mol-%, jeweils bezogen auf das gesamte Amingemisch.

Bevorzugt umfasst das Amingemisch zusätzlich ein Alkylendiamin, das besonders bevorzugt eine Verbindung der allgemeinen Formel

NH₂(CH₂)ₙNH₂

in welcher n eine ganze Zahl zwischen 2 und 10, bevorzugt 4 oder 6, ist. Bevorzugt einzusetzende Alkylendiamine tragen endständige Aminogruppen. Speziell bevorzugt ist das Hexamethylendiamin (Hexan-1,6-diamin). Das Alkylendiamin kann in der Polykondensationsreaktion in Mischung mit weiteren Diaminen oder Triaminen, wobei wenigstens ein Amin ausgewählt ist aus der Gruppe, die aus
i) Diamin, das wenigstens einen cycloaliphatischen Rest aufweist, und
ii) Dialkylentriamin besteht,
bevorzugt ausgewählt aus der Gruppe bestehend aus 4,4'-Methylenbis(cyclohexylamin) und/oder Diethylentriamin unter Ausbildung von Copolymeren eingesetzt werden.

Vorzugsweise kann das Amingemisch weiterhin Oxyalkylendiamine umfassen.

Als Oxyalkylendiamine eignen sich insbesondere solche Oxyalkylendiamine, die endständige Aminogruppen aufweisen. Ein bevorzugtes Oxyalkylendiamin ist eine Verbindung der allgemeinen Formel

NH₂[(CH₂)₂O)]ₙ(CH₂)₂NH₂

in welcher n eine ganze Zahl zwischen 2 und 6, bevorzugt zwischen 2 und 5, weiter bevorzugt zwischen 2 und 4 und insbesondere 2 ist. Bevorzugt sind Polyoxyethylendiamine, im Speziellen Triethylenglycoldiamin. Weiter bevorzugt eingesetzt werden können Polyoxypropylendiamine, insbesondere Di- oder Tripropylenglycoldiamin.

In einer bevorzugten Ausführungsform liegt der polymere oder oligomere Wirkstoff als Homopolymer vor. In diesen Fällen besteht das Amingemisch aus einem Diamin, das wenigstens einen cycloaliphatischen Rest aufweist, oder aus einem Dialkylentriamin.

In einer weiteren bevorzugten Ausführungsform besteht das Amingemisch aus dem Triamin Diethylentriamin. In dieser Variante liegt der polymere oder oligomere Wirkstoff somit als Homopolymer vor, beispielsweise als Poly(iminoimidazol).

In einer weiteren bevorzugten Ausführungsform besteht das Amingemisch aus dem Diamin 4,4'-Methylenbis(cyclohexylamin). Durch Polykondensation mit einem Guanidinsäureadditionssalz entsteht daraus beispielsweise das Homopolymer Poly(4,4'-methylenbis(cyclohexylaminhydrochlorid)).

Besonders bevorzugt sind die polymeren oder oligomeren Wirkstoffe, die erhältlich sind durch Polykondensation eines Guanidinsäureadditionssalzes mit einem Amingemisch, welches wenigstens ein Diamin, das wenigstens einen cycloaliphatischen Rest aufweist, umfasst. Diamine, die wenigstens einen cycloaliphatischen Rest aufweisen, sind beispielsweise cycloaliphatische Diamine, beispielsweise Cyclohexandiamin, Cyclopentandiamin sowie Derivate hiervon. Insbesondere bevorzugt sind solche Diamine, bei denen wenigstens eine NH₂-Gruppe direkt mit dem cycloaliphatischen Rest verknüpft ist. Besonders bevorzugt sind solche Diamine, bei denen beide NH₂-Gruppen jeweils direkt mit ein und demselben cycloaliphatischen Rest oder an verschiedenen cycloaliphatischen Resten verknüpft sind. In einer besonderen Ausführungsform umfasst das Amingemisch 4,4'-Methylenbis(cyclohexylamin).

In einer weiteren Ausführungsform der vorliegenden Erfindung umfasst das Amingemisch wenigstens ein Dialkylentriamin. Die Dialkylentriamine können dabei Alkylenreste unterschiedlicher Kettenlänge aufweisen. Bevorzugt sind allerdings Dialkylentriamine, bei denen die Alkylengruppen die gleiche Länge aufweisen. Bevorzugte Alkylenreste sind dabei Ethylen, Propylen und Butylen sowie Hexylen. In einer besonders bevorzugten Ausführungsform umfasst das Amingemisch das Triamin Diethylentriamin.

In einer weiteren bevorzugten Ausführungsform liegen die erfindungsgemäß verwendeten polymeren oder oligomeren Wirkstoffe (Komponente a)) in Form von Copolymeren vor. Diese können dabei sowohl willkürlich gemischt als auch als Blockcopolymere vorliegen. Im Falle von Copolymeren enthält das Amingemisch wenigstens zwei verschiedene Amine. Das Amingemisch enthält dabei eine erste Komponente und wenigstens eine zweite Komponente, wobei bevorzugt
die erste Komponente ein Diamin oder ein Triamin ist, das ausgewählt ist aus der Gruppe, die aus
   i) Diamin, das wenigstens einen cycloaliphatischen Rest aufweist, und
   ii) Dialkylentriamin besteht, und wobei
die zweite Komponente ein Diamin oder ein Triamin ist, das ausgewählt ist aus der Gruppe, die aus
   i) Diamin, das wenigstens einen cycloaliphatischen Rest aufweist,
   ii) Dialkylentriamin,
   iii) Alkylendiamin und
   iv) Oxyalkylendiamin besteht, und
wobei die erste Komponente von der zweiten Komponente verschieden ist.

Als besonders geeignete copolymere oder cooligomere Wirkstoffe haben sich solche herausgestellt, bei denen die erste Komponente 4,4'-Methylenbis(cyclohexylamin) ist und die zweite Komponente ausgewählt ist aus Diethylentriamin, Hexamethylendiamin und Triethylenglycoldiamin.

In einer weiteren bevorzugten Ausführungsform enthält das copolymere Guanidinderivat als erste Komponente Diethylentriamin und die zweite Komponente ist ausgewählt aus der Gruppe bestehend aus Hexamethylendiamin und Triethylenglycoldiamin.

Die erfindungsgemäße Öl in Wasser Emulsion enthält besonders bevorzugt eine Zusammensetzung, die einen antimikrobiellen Wirkstoff (Komponente a)) mit einem mittleren Molekulargewicht im Bereich von 500 bis 7000, insbesondere 1000 bis 5000 Dalton aufweist.

Im Rahmen der vorliegenden Erfindung wird der Begriff polymeres Guanidinderivat oder Bigunaid für Guanidinderivate bzw. Biguanide verwendet in denen 2 oder mehr Repetitionseinheiten auftreten. Der Begriff "Polymer" erfasst somit auch bereits Dimere, Trimere oder beispielsweise Oligomere.

Eine weitere Klasse polymerer Guanidinderivate ist beispielsweise in der WO-A1-01/85676 sowie in der WO-A1-06/047800 beschrieben.

Bevorzugte antimikrobielle Wirkstoffe (Komponente a)) sind dabei erhältlich durch Polykondensation eines Guanidin-Säureadditionssalzes mit einem Amingemisch, das wenigstens ein Diamin enthält, welches ausgewählt ist aus der Gruppe bestehend aus Aklylendiamin und Oxyalkylendiamin.

Bevorzugt umfasst hierbei das Amingemisch ein Alkylendiamin, das besonders bevorzugt eine Verbindung der allgemeinen Formel

NH₂(CH₂)ₙNH₂

in welcher n eine ganze Zahl zwischen 2 und 10, bevorzugt 4 oder 6, ist. Bevorzugt einzusetzende Alkylendiamine tragen endständige Aminogruppen. Speziell bevorzugt ist das Hexamethylendiamin (Hexan-1,6-diamin). Das Alkylendiamin kann in der Polykondensationsreaktion in Mischung mit weiteren Polyaminen, beispielsweise Di- und/oder Triaminen unter Ausbildung von Copolymeren eingesetzt werden.

Vorzugsweise umfasst das Amingemisch wenigstens ein Oxyalkylendiamin.

Als Oxyalkylendiamine eignen sich insbesondere solche Oxyalkylendiamine, die endständige Aminogruppen aufweisen. Ein bevorzugtes Oxyalkylendiamin ist eine Verbindung der allgemeinen Formel

NH₂[(CH₂)₂O)]ₙ(CH₂)₂NH₂

in welcher n eine ganze Zahl zwischen 2 und 6, bevorzugt zwischen 2 und 5, weiter bevorzugt zwischen 2 und 4 und insbesondere 2 ist. Bevorzugt sind Oxyethylendiamine, im Speziellen Diethylenglycoldiamin oder Triethylenglycoldiamin. Weiter bevorzugt eingesetzt werden können Polyoxypropylendiamine, insbesondere Di- oder Tripropylenglycoldiamin.

Bevorzugt liegt das polymere Guanidinderivat als Homopolmyer vor. In diesen Fällen besteht das Amingemisch aus dem Alkylendiamin oder aus einem Oxyalkylendiamin.

In einer weiteren bevorzugten Ausführungsform besteht das Amingemisch aus dem Alkylendiamin Hexamethylendiamin (Hexan-1,6-diamin). In dieser Variante besteht das polymere Guanidinderivat somit aus einem Homopolymer, beispielsweise das Poly-(hexamethylen-guanidinium-chlorid) (PHMG).

In einer weiteren bevorzugten Ausführungsform besteht das Amingemisch aus dem Oxyalkylendiamin Triethylenglycoldiamin. Durch Polykondensation mit einem Guanidinsäureadditionssalz entsteht daraus beispielsweise das Homopolymer Poly-[2-(2-ethoxy)-ethoxyethyl)-guanidinium-chlorid].

In einer weiteren bevorzugten Ausführungsform liegen die erfindungsgemäß verwendeten polymeren Guanidinderivate in Form von Copolymeren vor. Diese können dabei sowohl willkürlich gemischt als auch als Blockcopolymere vorliegen. Im Falle von Copolymeren enthält das Amingemisch wenigstens zwei verschiedene Amine. Das Amingemisch enthält dabei eine erste Komponente und wenigstens eine zweite Komponente, wobei
- die erste Komponente ein Diamin ist, ausgewählt aus der Gruppe bestehend aus Alkylendiamin und Oxyalkylendiamin, und wobei
- die zweite Komponente ein Diamin ist, das ausgewählt aus der Gruppe bestehend aus Alkylendiamin und Oxyalkylendiamin, und
wobei die erste Komponente von der zweiten Komponente verschieden ist.

Als besonders geeignete copolymere Guanidinderivate haben sich solche herausgestellt, bei denen die erste Komponente Alkylendiamin und die zweite Komponente ein Oxyalkylendiamin ist. Besonders bevorzugt sind copolymere Guanidinderivate bei denen im Amingemisch die erste Komponente Hexamethylendiamin und die zweite Komponente Triethylenglycoldiamin ist.

Bei der Herstellung von Copolymeren kann das Mischungsverhältnis der einzusetzenden Amine in weiten Bereichen variiert werden. Bevorzugt sind allerdings copolymere Guanidinderivate, bei denen die Monomere des Amingemischs, also die erste Komponente und die zweite Komponente, in einem Molverhältnis von 4 : 1 bis 1 : 4, vorzugsweise 2 : 1 bis 1 : 2, vorliegt.

Die erfindungsgemäß einzusetzenden polymeren Guanidinderivate besitzen bevorzugt ein mittleres Molekulargewicht (Gewichtsmittel) im Bereich von 500 bis 7000, insbesondere von 1000 bis 5000 Dalton.

In einer weiteren bevorzugten Ausführungsform der vorliegenden Erfindung liegt das erfindungsgemäß einzusetzende polymere Guanidinderivat als Mischung aus mindestens 2 unterschiedlichen polymeren Guanidinderivaten vor. In einer speziellen Ausführungsform umfasst die Mischung der polymeren Guanidinderivate sowohl ein erstes Homopolymer basierend auf einem Alkylendiamin, vorzugsweise Poly-(hexamethylen-guanidinium-chlorid) als auch ein zweites Homopolymer basierend auf einem Oxyalkylendiamin, beispielsweise Poly-[2-(2-ethoxy)-ethoxyethyl)-guanidinium-chlorid].

In einer bevorzugten Ausführungsform umfasst das polymere Guanidinderivat das erste Homopolymer und das zweite Homopolymer in einem Gewichtsverhältnis von 5:1 bis 1:5, vorzugsweise 1:1 bis 1:4 und insbesondere 1:2 bis 1:4. In einer besonders bevorzugten Ausführungsform umfasst das polymere Guanidingemisch Poly-(hexamethylen-guanidinium-chlorid) (erstes Homopolymer) und Poly-[2-(2-ethoxy)-ethoxyethyl)-guanidinium-chlorid] (zweites Homopolymer) in einem Gewichtsverhältnis (erstes Homopolymer zu zweitem Homopolymer) von 1:1 bis 1:5, vorzugsweise 1:2 bis 1:4, insbesondere 1:3. Solche Mischungen eignen sich insbesondere für die Öl in Wasser Emulsion der vorliegenden Erfindung.

Die erfindungsgemäß verwendeten polymeren Guanidinderivate und Biguanide können allgemein sowohl als Homopolymer als auch als Copolymer vorliegen. Vorteilhaft ist dabei, wenn das Guanidin-Säureadditionssalz Guanidiniumhydrochlorid ist. Es sind aber auch weitere Guanidin-Säureadditionssalze, auf Basis anorganischer oder organischer Säuren ebenfalls geeignet. Beispielsweise die Hydroxide, Hydrogensulfate sowie Acetate. Besonders geeignete und wirksame polymere Guanidinderivate liegen in Form ihrer Hydroxid-Salze vor. Diese können beispielsweise durch Anionenaustausch aus den korrespondierenden Chlorid-Salzen erhalten werden.

Die Komponente a) liegt in den erfindungsgemäßen Öl in Wasser Emulsionen üblicherweise in einer Menge von 0,005 bis 0,2 Gew.-%, vorzugsweise 0,01 bis 0, 19 Gew.-%, insbesondere 0,02 bis 0,1 Gew-% und im Speziellen 0,03 bis 0,07 Gew.-%, jeweils bezogen auf das Gesamtgewicht der Emulsion, vor. Insbesondere bezüglich der verminderten Zytotoxizität hat es sich als vorteilhaft herausgestellt, das Gewichtsverhältnis von Komponente a) zu Komponente b) aufeinander abzustimmen. In der vorliegenden Ausführungsform ist das Gewichtsverhältnis von Komponente a) zu Komponente b) 1:4 bis 1:40, vorzugsweise 1:6 bis 1:20 und insbesondere 1:8 bis 1:12.

Weiterhin ist es vorteilhaft, insbesondere im Hinblick auf die Ausbildung geeigneter Öl in Wasser Emulsionen, die die Bedingungen für geringe Zytotoxizität erfüllen, dass das Gewichtsverhältnis von Komponente a) zur Komponente c) (Ölkomponente) 1:80 bis 1:1000, vorzugsweise 1:100 bis 1:800, weiter bevorzugt 1:150 bis 1:600 und insbesondere 1:180 bis 1:450 liegt.

Es wurde weiter überraschend gefunden, dass die Partikelgröße der emulgierten Öltröpfchen, insbesondere bei der Behandlung von Haut, vorteilhafter Weise im nanoskaligen Bereich einzustellen ist. In einer bevorzugten Ausführungsform der vorliegenden Erfindung weisen die emulgierten Öltröpfchen eine mittlere Partikelgröße von bis zu 300 nm, vorzugsweise 30 bis 260 nm, weiter bevorzugt 50 bis 100 nm auf. Die Bestimmung der Teilchengröße erfolgt mit Photonenkorrelationsspektroskopie (Autosizer II Malvern Instruments UK) bei 20°C. Insbesondere die nanopartikulären Öl in Wasser Emulsion scheinen überraschend in die Haarfollikel einzudringen. Hier hat sich überraschend gezeigt, dass die starren Nanopartikel der vorliegenden Öl in Wasser Emulsion wesentlich besser in die Haarfollikel transportiert werden können, als beispielsweise Liposomen.

Ohne an eine Theorie gebunden zu sein, scheint der Wirkmechanismus der starren mit Phospholipiden emulgierten Öltröpfchen offensichtlich darauf zu beruhen, dass das sich bewegende Haar die partikulären Substanzen wie eine Zahnradpumpe in die Haarfollikel transportiert. Dabei kommt den Haarschuppen große Bedeutung zu. Handelt es sich bei den Nanopartikeln um relativ starre Teilchen, wie in der vorliegenden Erfindung, so können die Haarschuppen diese Partikel besser erfassen und effektiver in den Haarfollikel transportiert werden. Bei herkömmlichen Liposomen, die eine flexible Struktur aufweisen, ist dieser Transportprozess schwierig. Außerdem werden Liposomen beim Eindringen in die Haut zerstört.

Weiterhin können die erfindungsgemäßen Emulsionen weitere Zusatzstoffe enthalten. Bevorzugt enthalten die erfindungsgemäßen Emulsionen zusätzlich ein Isotonisierungsmittel, beispielsweise Glycerin. Das Isotonisierungsmittel liegt üblicherweise in einer Menge von bis zu 3 Gew.-%, vorzugsweise 0,5 bis 2,5 Gew.-%, weiter bevorzugt 1 bis 1,5 Gew.-%, jeweils bezogen auf das Gesamtgewicht der Emulsion, vor.

Weiterhin bevorzugt enthalten die erfindungsgemäßen Emulsionen ein pH-Wert-Regulierungsmittel, beispielsweise Natriumoleat. Die pH-Wert-Regulierungsmittel liegen bevorzugt in einer Menge von bis zu 0,5 Gew-%, weiter bevorzugt in einer Menge von 0,1 bis 0,3 Gew.-%, jeweils bezogen auf das Gesamtgewicht der Emulsion, vor.

Zur weiteren Stabilisierung der Öl in Wasser Emulsion können die erfindungsgemäßen Emulsionen weiterhin zusätzlich ein Antioxidans, beispielsweise α-Tocopherol enthalten. Üblicherweise werden die Antioxidantien in einer Menge von bis zu 0,3 Gew.-%, vorzugsweise 0,05 bis 0,2 Gew.-%, jeweils bezogen auf das Gesamtgewicht der Emulsion, eingesetzt.

Zur Wirkungsverstärkung können die erfindungsgemäßen Öl in Wasser Emulsionen weiterhin zusätzlich eine lipophile Substanz mit synergistischer antimikrobieller Wirkung enthalten. Hierzu eignen sich insbesondere ätherische Öle.

In einer besonders bevorzugten Ausführungsform enthalten die erfindungsgemäßen Öl in Wasser Emulsionen zusätzlich ein oder mehrere Alkandiol(e).

Es wurde überraschend gefunden, dass Alkandiole die Wundheilung fördern und sogar zu einer Verstärkung der Wirksamkeit gegen bestimmte Keime, wie beispielsweise Hefen (Candida) beitragen. Darüber hinaus wurde festgestellt, dass durch Einsatz von Alkandiolen in den erfindungsgemäßen Öl in Wasser Emulsionen die Konzentration der antimikrobiellen Wirkstoffe (Komponente a)) abgesenkt werden kann bei gleichbleibender antimikrobieller Wirkung im Vergleich zu Emulsionen ohne Alkandiolen. Dies führt zusätzlich zu einer weiteren Herabsetzung der Cytotoxizität.

Als geeignete Alkandiole haben sich insbesondere solche herausgestellt, die 3 bis 12 Kohlenstoffatome aufweisen. Besonders bevorzugt sind Alkandiole ausgewählt aus der Gruppe bestehend aus 1,2 Propylenglykol, 1,3-Butylenglykol, 1,2-Pentandiol, 1,2-Hexandiol, 1,6-Hexandiol und/oder 1,2-Octandiol. Bevorzugt sind 1,2-Alkandiole mit 5 bis 10 Kohlenstoffatomen, insbesondere 1,2 Octandiol.

Die Alkandiole werden bevorzugt in Mengen von 0,01 bis 10 Gew.-%, insbesondere 0,05 bis 5 Gew.-%, jeweils bezogen auf das Gesamtgewicht Öl in Wasser Emulsion, eingesetzt.

Die erfindungsgemäßen Emulsionen können bevorzugt als Antiseptikum, insbesondere für Wunden und Körperhöhlenspülungen eingesetzt werden. Es ist daher von Vorteil, den pH-Wert der erfindungsgemäßen Emulsion in einen Bereich von 5 bis 9, vorzugsweise 5,5 bis 7 und insbesondere zwischen 6 und 7 einzustellen.

Ein weiterer Gegenstand der vorliegenden Erfindung ist eine pharmazeutische Zubereitung, die die erfindungsgemäße antimikrobielle Öl in Wasser Emulsion umfasst bzw. aus ihr besteht.

Ein weiterer Gegenstand der vorliegenden Erfindung ist ein Antiseptikum, welches die erfindungsgemäße Öl in Wasser Emulsion umfasst.

In einer bevorzugten Ausführungsform der vorliegenden Erfindung wird das erfindungsgemäße Antiseptikum zur Prophylaxe oder Behandlung der Haut, der Schleimhaut, des Genitalbereichs, des Auges, sowie von Wunden eingesetzt. Ein bevorzugter Gegenstand ist daher das erfindungsgemäße Antiseptikum zur Verwendung in der Prophylaxe oder Behandlung von Infektionen der Haut, der Schleimhaut, des Genitalbereichs, des Auges, sowie von Wunden.

Insbesondere geeignet sind die Antiseptika der vorliegenden Erfindung für Körperhöhlenspülungen, Gelenkspülungen, als Augenantiseptikum, als Bestandteil von Wundauflagen, als Wundantiseptikum, zur Nasenhöhlenantiseptik, insbesondere bei MRSA-Besiedlung, zur Genital- und Mundhöhlenantiseptik sowie zur Hautantiseptik.

Die pharmazeutische Zubereitung der vorliegenden Erfindung ist besonders geeignet zur Prophylaxe und Therapie von Infektionen, insbesondere von Infektionen an empfindlichen Körperregionen, wie beispielsweise dem Peritoneum, der Bauchhöhle, der Harnblase, des Harnleiters, des zentralen Nervensystems und hyalinem Gelenkknorpel.

Darüber hinaus werden die erfindungsgemäßen pharmazeutischen Zubereitungen zur Prophylaxe oder Therapie atopischer Dermatiden, infizierter Ekzeme und von Dermatomykosen eingesetzt. Ein bevorzugter Gegenstand ist daher die erfindungsgemäße pharmazeutische Zubereitung zur Verwendung in der Prophylaxe oder Therapie atopischer Dermatiden, infizierter Ekzeme und von Dermatomykosen.

Ein weiterer Gegenstand der vorliegenden Erfindung ist die Verwendung der erfindungsgemäßen Emulsion zur Wundbehandlung, beispielsweise zur Herstellung einer Wundauflage, die insbesondere fest oder gelartig ist. In einer besonderen Ausführungsform wird hierzu das Trägermaterial der Wundauflage mit der erfindungsgemäßen Emulsion berieselt, besprüht oder imprägniert oder in eine gelartige Wundauflagen eingearbeitet.

Ein weiterer Gegenstand der vorliegenden Erfindung ist eine Wundauflage, die die erfindungsgemäße antimikrobielle Öl in Wasser Emulsion umfasst. Als Trägermaterialien für die Herstellung von Wundauflagen kommen alle dafür gebräuchlichen und dem Fachmann geläufigen Materialien in Betracht, z.B. Collagen, Cellulosen und Cellulosederivate, Polyurethane, Alginate, allein oder in Kombination mit Polysacchariden aus der Gruppe, die aus Alginaten, Glycosaminoglycanen und ihren Salzen, Pektinen, Carrageenanen, Xanthanen, sulfatierten Dextranen, Cellulosederivaten, oxidierter Cellulose, wie oxidierter, regenerierter Cellulose, Stärkederivate und deren Mischungen.

In einer bevorzugten Ausführungsform wird die erfindungsgemäße Emulsion auf eine schwammartige Trägersubstanz aufgebracht.

Die vorliegende Erfindung bezieht sich daher auch auf Wundauflagen, die die erfindungsgemäße Emulsion umfasst und beispielsweise auf Cellulose, einem Cellulosederivat wie Carboxymethylcellulosen, Alginaten, Chitosan, Stärke oder Stärkederivaten, Collagen, Polyacrylaten, Polyurethan oder Mischungen der vorgenannten Verbindung als Trägermaterial basiert.

Die bevorzugten Ausführungsformen der erfindungsgemäßen Wundauflagen sind Hydrogele, Hydrocolloide, Schwämme, Folien, Membranen, Fliese, Gewebe, Gewirke, andere textile Flächengebilde, Kardenbänder, Tamponaden und dergleichen.

Ein weiterer Gegenstand der vorliegenden Erfindung ist ein Verfahren zur Herstellung der erfindungsgemäßen Emulsion umfassend die Schritte:
α) Herstellung einer Öl in Wasser Emulsion umfassend
   b) mindestens ein Phospholipid und
   c) mindestens einer Ölkomponente und
β) Vermischen der in Schritt α) hergestellten Emulsion mit einer wässrigen Lösung umfassend
   a) mindestens eine aliphatischen Komponente, die eine oder mehrere Guanid- und/oder Biguanid-Gruppe(n) aufweist,
wobei das Gewichtsverhältnis von Komponente a) zu Phospholipid 1:4 bis 1:40 beträgt und die Komponente a) in der Emulsion in einer Menge von 0,005 bis 0,2 Gew.-%, bezogen auf das Gesamtgewicht der Emulsion, vorliegt.

Bezüglich des erfindungsgemäßen Verfahrens und dem Einsatz der jeweiligen Komponenten wird auf die vorgehend genannten Ausführungsformen der erfindungsgemäßen Emulsion voll umfänglich Bezug genommen. Die dort genannten bevorzugten Ausführungsformen gelten auch für das erfindungsgemäße Verfahren.

In einer bevorzugten Ausführungsform des erfindungsgemäßen Verfahrens, schließt sich an Schritt a) oder Schritt b) ein weiterer Schritt an, nämlich die Hochdruckhomogenisierung. Mittels Hochdruckhomogenisierung lassen sich einfach erfindungsgemäße Öl in Wasser Emulsionen herstellen, die Partikelgrößen mit einer mittleren Partikelgröße unterhalb von 300 nm aufweisen, erhalten.

Die nachfolgenden Beispiele erläutern die Erfindung.

### Beispiel 1

### Herstellung einer erfindungsgemäßen Öl in Wasser Emulsion

Es wird zunächst eine Öl in Wasser Emulsion durch Homogenisieren der folgenden in der Tabelle 1 aufgeführten Komponenten bereitgestellt:

**Tabelle 1**

| Menge in Gewichtsprozent (Gew.-%) | Komponente |
|---|---|
| 10 Gew.-% | mittelkettiges Triglycerid¹⁾ |
| 10 Gew.-% | Sojabohnenöl |
| 2,25 Gew.-% | Glycerin |
| 1,2 Gew.-% | Eilecithin |
| 0,25 Gew.-% | Natriumoleat |
| 0,2 Gew.-% | α -Tocopherol |
| ad 100 | Wasser |

| | |
|---|---|
| ¹⁾ Glycerin, welches mit Fettsäuren verestert ist, die bis zu 98 mol.-% aus Caprylsäure (C₈) und Caprinsäure (C₁₀) bestehen. | |

Die in Tabelle 1 aufgeführte Öl in Wasser Emulsion wird mit dem gleichen Volumen einer Lösung, die 0,1 Gew.-% Polyhexamethylenbiguanidiniumhydrochlorid in Wasser aufweist, gemischt.

Die daraus resultierende erfindungsgemäße Öl in Wasser Emulsion ist sofort einsatzfähig.

### Beispiel 2

### Herstellung einer erfindungsgemäßen Öl in Wasser Emulsion

Es wird zunächst eine Öl in Wasser Emulsion durch Homogenisieren der folgenden in der Tabelle 2 aufgeführten Komponenten bereitgestellt:

**Tabelle 2**

| Menge in Gewichtsprozent (Gew.-%) | Komponente |
|---|---|
| 5 Gew.-% | mittelkettiges Triglycerid¹⁾ |
| 5 Gew.-% | Sojabohnenöl |
| 2,25 Gew.-% | Glycerin |
| 1,2 Gew.-% | Eilecithin |
| 0,25 Gew.-% | Natriumoleat |
| 0,2 Gew.-% | α-Tocopherol |
| ad 100 | Wasser |

| | |
|---|---|
| ¹⁾ Glycerin, welches mit Fettsäuren verestert ist, die bis zu 98 mol.-% aus Caprylsäure (C₈) und Caprinsäure (C₁₀) bestehen. | |

Die in Tabelle 2 aufgeführte Öl in Wasser Emulsion wird mit dem gleichen Volumen einer Lösung, die 0,1 Gew.-% Polyhexamethylenbiguanidiniumhydrochlorid in Wasser aufweist, gemischt.

Die daraus resultierende erfindungsgemäße Öl in Wasser Emulsion ist sofort einsatzfähig.

Die Konzentration des Polihexanids in Beispiel 1 und Beispiel 2 beträgt somit 0,05%.

Als Vergleichsbeispiel für die nachfolgenden Untersuchungen, wird eine wässrige Lösung enthaltend 0,05% Polihexanid verwendet.

### I. In-vitro-Resultate zur antimikrobiellen Wirkung und zur Zytotoxizität zum Ausführungsbeispiel 1) und 2)

Die Mikrobiozidie-Prüfung erfolgte im quantitativen Suspensionstest nach DIN EN 1040 (2006) bei Raumtemperatur, wobei sowohl die Prüfpräparate als auch die Prüfmikroorganismen in MEM-Zellkulturmedium mit 10 % fötalem bovinen Serum (FBS) eingebettet wurden, damit die gleichen Konditionen sowohl für die Mikrobiozidie-Prüfung als auch für die Bestimmung der Zytotoxizität vorhanden sind. Dazu wurden gleiche Volumina Prüfpräparat und doppelt konzentriertes Zellkulturmedium gemischt. Aus methodischen Gründen können die in Zellkulturmedium eingebetteten Prüfpräparate nur in Konzentrationen ≤ 50 % geprüft werden.

### Prüfmikroorganismen

| | |
|---|---|
| Pseudomonas aeruginosa | ATCC 15442 |
| Staphylococcus aureus | ATCC 6538 |

**Tabelle 3: Ergebnisse Mikrobiozidie:**

| Zubereitung | PHMB-Endkonzentration [µg/ml] | Ig Reduktionsfaktor | | | |
|---|---|---|---|---|---|
| | | *P. aeruginosa* | | *S. aureus* | |
| | | 30 min | 60 min | 30 min | 60 min |
| Vergleichsbeispiel | 225 | > 3 | > 3 | > 3 | > 3 |
| Beispiel 2 | 225 | > 3 | > 3 | > 3 | > 3 |
| Beispiel 1 | 225 | > 3 | > 3 | > 3 | > 3 |

Die unmittelbar nach Herstellung der Präparate erzielten Prüfergebnisse des quantitativen Suspensionstests sind nach 6 - 8 Monaten Lagerung reproduzierbar.
II. Für die Zytotoxizitäts-Untersuchungen wurden Mäusefibroblasten der Zell-Linie L929 (ATCC CCL 1) als Testzellen verwendet. Die Prüfungen erfolgten nach DIN EN ISO 10993-5 (1999) bei 20°C, wobei die Prüfpräparate in MEM-Zellkulturmedium mit 10 % FBS eingebettet wurden, damit die gleichen Konditionen sowohl für die Mikrobiozidie-Prüfung als auch für die Bestimmung der Zytotoxizität vorhanden sind. Dazu wurden gleiche Volumina Prüfpräparat und doppelt konzentriertes Zellkulturmedium gemischt. Die Auswertung erfolgte mit Hilfe von zwei verschiedenen Vitalitätsprüfungen, der Neutralrot (NR)-Methode und dem MTT-Test.

Bei einer Endkonzentration von 250 µg/ml PHMB in der Vergleichszubereitung waren nach 30 min Einwirkung nur noch 10 % der Mäusefibroblasten vital, wohingegen sämtliche vergleichbare PHMB-Konzentrationen der erfindungsgemäßen Zubereitungen keine Zytotoxizität aufwiesen. Prüfzelle

| | |
|---|---|
| Mäusefibroblasten (L929-Zellen) | ATCC CCL 1 |

**Tabelle 4: Ergebnisse Zytotoxizität:**

| Zubereitung | PHMB-Endkonzentration [µg/ml] | L929-Zell-Vitalität [%] | | | |
|---|---|---|---|---|---|
| | | MTT-Test | | N R-Test | |
| | | 30 min | 60 min | 30 min | 60 min |
| Vergleichsbeispiel | 250 | 13 | 11 | 8 | 6 |
| Beispiel 2 | 250 | 115 | 110 | 94 | 88 |
| Beispiel 1 | 250 | 110 | 120 | 95 | 95 |

Die unmittelbar nach Herstellung der Präparate erzielten Prüfergebnisse der In-vitro-Zytotoxizitäts-Untersuchungen sind nach 6 - 8 Monaten Lagerung reproduzierbar.

Die Ergebnisse in den beiden Tabellen 3 und 4 zeigen, dass die mikrobiozide Wirkung gegen beide Mikroorganismen bei beiden Ausführungsbeispielen gleich gut ist wie für die reine PHMB-Lösung (siehe Tabelle 3). Damit werden die Wirkungsanforderungen für Antiseptika unter Belastung erfüllt. Die in der PHMB-Lösung gemessene Zytotoxizität hingegen ist in den Ausführungsbeispielen 1 und 2 nahezu komplett verschwunden (siehe Tabelle 4).
III. Die Mikrobiozidie und die Zytotoxizität wurden außerdem in einem Kombinationstest untersucht. Dazu wurden Mäusefibroblasten der Zell-Linie L929 (ATCC CCL 1) als Testzellen verwendet und mit 9 Volumeneinheiten 10⁶ KbE Prüf-Mikroorganismen (Prüf-MO) *Pseudomonas aeruginosa* bzw. *Staphylococcus aureus* infiziert, die in MEM-Zellkulturmedium mit 10 % FBS eingebettet wurden. Anschließend erfolgte die Zugabe von 1 Volumeneinheit der Zubereitung. Nach 30 min Kontakt der Zubereitung sowohl nur mit den Prüfzellen als auch mit den Kombinationen aus Prüfzellen und Prüf-MO bei Raumtemperatur erfolgte die Bestimmung des Reduktionsfaktors mittels quantitativem Suspensionstest und die Nachweisreaktion für die Vitalität der L929-Zellen mittels NR-Test.

### Prüfzelle

| | |
|---|---|
| Mäusefibroblasten (L929-Zellen) | ATCC CCL 1 |

### Prüfmikroorganismen

| | |
|---|---|
| *Pseudomonas aeruginosa* | ATCC 15442 |
| *Staphylococcus aureus* | ATCC 6538 |

**Tabelle 5: Ergebnisse Mikrobiozidie/Zytotoxizität:**

| Zubereitung | PHMB-Konz. [µg/ml] | Mikrobiozidie Ig Reduktionsfaktor | | Zytotoxizität (NR-Test) L929-Zell-Vitalität [%] | |
|---|---|---|---|---|---|
| | | *P. aeruginosa* | *S. aureus* | mit Prüf-MO | ohne Prüf-MO |
| Vergleichsbeispiel | 50 | > 3 | > 3 | 18 | 16 |
| Beispiel 2 | 50 | > 3 | > 3 | 97 | 97 |
| Beispiel 1 | 50 | > 3 | > 3 | 99 | 97 |

Die Ergebnisse in Tabelle 5 zeigen, dass die mikrobiozide Wirkung von PHMB in den Zubereitungen mit der der Vergleichszubereitung in Wasser identisch ist. Die zytotoxische Wirkung auf die L929-Zellen ist nur für die Vergleichszubereitung nachweisbar, nicht jedoch für die Zubereitung von PHMB in Form der Öl in Wasser Emulsion.

### IV. In-vivo Resultate zum Ausführungsbeispiel 2)

Im Rahmen einer klinischen Studie wurde die Wirkung der erfindungsgemäßen Zubereitung von PHMB in Form der Öl in Wasser Emulsion mit einer wässrigen PHMB-Lösung verglichen. Die Untersuchungen erfolgten am Unterarm von gesunden Probanden. Vor und nach Hautantiseptik wurden von den jeweiligen Hautarealen Abstriche entnommen und diese bakteriologisch untersucht. Bei Gabe gleicher Mengen von PHMB zeigte es sich, dass bei Anwendung der Öl in Wasser Emulsion eine signifikant bessere antiseptische Wirkung (Faktor 2) erzielt werden konnte als nach Antiseptik mit der wässrigen Lösung von Polihexanid (Vergleichsbeispiel).

Nach 1 h konnte die bakterielle Besiedlung beim Einsatz des Antiseptikum als Öl in Wasser Emulsion auf 20 % reduziert werden, während mit der wässrigen Lösung von PHMB nur eine Abnahme auf 40 % der ursprünglichen Besiedlung erzielt wurde. Nach 3 h betrug die bakterielle Besiedlung mit der partikulären Form 60 %, während die nicht partikuläre Form zu einer Besiedlung von 80 % im Vergleich zur unbehandelten Haut führte.

Dieses Ergebnis ist offensichtlich darauf zurückzuführen, dass die emulgierten Ölpartikel im Vergleich zum gelösten PHMB besser in die Haarfollikel eindringen. Das bewegte Haar scheint hierbei eine Art Pumpeffekt zu induzieren, der die Öltröpfchen in die Haarfollikel transportiert.

### V. Untersuchung der erfindungsgemäßen Öl in Wasser Emulsion in Bezug auf eine Peritoneallavage auf die abdominelle Sepsis in der Maus

Es wurden 4 Formulierungen untersucht:
i) Erfindungsgemäße Öl in Wasser Emulsion gemäß Beispiel 2
ii) Öl in Wasser Emulsion gemäß Tabelle 2, wobei die Emulsion mit dem gleichen Volumen Wasser verdünnt wird (Vergleichsemulsion)
iii) Wäßrige Lösung enthaltend 0,05 Gew.-% PHMB [Vergleichslösung]
iv) Wäßrige Lösung enthaltend 0,9 Gew.-% NaCl (physiologische Kochsalzlösung)[Vergleichslösung]

### Versuchsbeschreibung Peritoneallavage:

Um eine Peritonitis zu simulieren, wurde in einem von der Ethikkommission genehmigten Tierversuch an Mäusen eine polymikrobielle Sepsis induziert.
Dazu wurde den Versuchstieren in Vollnarkose ein Stent definierten Durchmessers in das Colon Ascendens implantiert. Es entstand eine Leckage definierter Größe, die durch den Stent offen gehalten wurde. Durch die Versuchsbedingungen kam es rasch zu einer systemischen Infektion und zum septischen Schock. Nach 6 Stunden wurde der Stent enfernt, die Darmwand verschlossen und die Mäuse in 4 Gruppen aufgeteilt. Jeder Gruppe wurde eine der Formulierungen i), ii), iii) und iv) zugeordnet, mit der die infizierten Areale gespült wurden. Die Überlebensrate der Mäuse wurden verglichen.

**Tabelle 6: Ergebnisse der Peritoneallavage**

| Formulierung | n (Anzahl Versuchstiere) | Überlebensrate |
|---|---|---|
| i) | 21 | 70 % |
| ii) | 23 | 20% |
| iii) | 21 | <10% |
| iv) | 20 | 40% |

Die Peritoneallavage mit der erfindungsgemäßen Öl in Wasser Emulsion führte zu einer deutlich höheren Überlebensrate der Versuchstiere als mit den Vergeichsformulierungen ii) bis iv).

### VI. Vergleich der Wirksamkeit von PHMB mit Chlorhexidin in Öl in Wasser Emulsionen

Es wurden Öl in Wasser Emulsionen hergestellt, die unterschiedliche Mengen X des antimikrobiellen Wirkstoffs Polyhexamethylenbiguanidiniumhydrochlorid (PHMB) [erfindungsgemäß] und Chlorhexidin (CHX) [Vergleich] aufweisen. Die Öl in Wasser Emulsionen sind in Tabelle 7 wiedergegeben.

**Tabelle 7**

| Menge in Gewichtsprozent (Gew.-%) | Komponente |
|---|---|
| 10 Gew.-% | mittelkettiges Triglycerid¹⁾ |
| 10 Gew.-% | Sojabohnenöl |
| 2,5 Gew.-% | Glycerin |
| 1,2 Gew.-% | Eilecithin |
| 0,03 Gew.-% | Natriumoleat |
| 0,02 Gew.-% | α-Tocopherol |
| x Gew.-% | Antimikrobieller Wirkstoff |
| ad 100 | Wasser |

| | |
|---|---|
| ¹⁾ Glycerin, welches mit Fettsäuren verestert ist, die bis zu 98 mol.-% aus Caprylsäure (C₈) und Caprinsäure (C₁₀) bestehen. | |

Die Menge x in Gew.-% und der jeweils eingesetzte Wirkstoff sind in der nachfolgenden Tabelle 8, linke Spalte wiedergegeben.

Um die Wirksamkeit der antimikrobiellen Wirkstoffe in einem bestimmten Konzentrationsbereich in einer Öl in Wasser Emulsion zu testen wurde ein Inaktivatortest nach prEN13727 (2008) und prEN13624 (2010) gegenüber *Enterococcus hirae* (ATCC 10541), *Escherichia coli* (ATCC 11229), *Pseudomonas aeruginosa* (ATCC 15442) und *Staphylococcus aureus* (ATCC 6538) für n = 4 durchgeführt. Die Messergebnisse sind in Tabelle 8 wiedergegeben.

**Tabelle 8**

| Endkonzentration Antimikrobieller Wirkstoff in o/w Emulsion | Kolonien-bildende Einheiten | | | |
|---|---|---|---|---|
| | *E. hirae* | *E. coli* | *P. aeruginosa* | *S. aureus* |
| Kontrolle (x=0 %) | 103 ± 5 | 138 ± 10 | 331 ± 30 | 169 ± 12 |
| 0,02% CHX | 104 ± 4 | 132 ± 1 | 316 ± 50 | 149 ± 5 |
| 0,03% CHX | 102 ± 5 | 131 ± 5 | 315 ± 43 | 156 ± 13 |
| 0,04% CHX | 106 ± 3 | 130 ± 9 | 355 ± 10 | 160 ± 8 |
| 0,05% CHX | 106 ± 3 | 130 ± 9 | 339 ± 37 | 159 ± 10 |
| Kontrolle (x=0 %) | 107 ± 7 | 132 ± 7 | 338 ± 36 | 180 ± 22 |
| 0,02% PHMB | 0 | 0 | 0 | 0 |
| 0,03% PHMB | 0 | 0 | 0 | 0 |
| 0,04% PHMB | 0 | 0 | 0 | 0 |
| 0,05% PHMB | 0 | 0 | 0 | 0 |

### Ergebnis :

Die Öl in Wasser Emulsion inaktiviert vollständig die Wirkung von Chlorhexidin (CHX) im untersuchten Konzentrationsbereich von 0,02 - 0,05 Gew.-%, wohingegen die mikrobiozide Wirkung von PHMB im vergleichbaren Konzentrationsbereich vollständig erhalten bleibt (keine KbE Prüfmikroorganismen mehr nachweisbar).

### VII. Öl in Wasser Emulsionen mit Alkandiol

### Beispiel 3: Erfindungsgemäße Öl in Wasser Emulsion mit Alkandiol

| Menge in Gewichtsprozent (Gew.-%) | Komponente |
|---|---|
| 10 Gew.-% | mittelkettiges Triglycerid¹⁾ |
| 10 Gew.-% | Sojabohnenöl |
| 2,25 Gew.-% | 1,2-Octandiol |
| 1,2 Gew.-% | Eilecithin |
| 0,25 Gew.-% | Natriumoleat |
| 0,2 Gew.-% | α-Tocopherol |
| 0,5 Gew.-% | PHMB (20 %) ²⁾ |
| ad 100 | Wasser |

| | |
|---|---|
| ¹⁾ Glycerin, welches mit Fettsäuren verestert ist, die bis zu 98 mol.-% aus Caprylsäure (C₈) und Caprinsäure (C₁₀) bestehen ²⁾ wässrige Lösung enthaltend 20 Gew.-% Polyhexamethylenbiguanid Hydrochlorid (PHMB) | |

### Beispiel 4: Erfindungsgemäße Öl in Wasser Emulsion mit Alkandiol

| Menge in Gewichtsprozent (Gew.-%) | Komponente |
|---|---|
| 20 Gew.-% | Triisostearin |
| 2,25 Gew.-% | 1,2-Octandiol |
| 1,2 Gew.-% | Hydriertes Sojalecithin |
| 0,25 Gew.-% | Natriumisostearat |
| 0,5 Gew.-% | PHMB (20 %) ¹⁾ |
| ad 100 | Wasser |

| | |
|---|---|
| ¹⁾ wässrige Lösung enthaltend 20 Gew.-% Polyhexamethylenbiguanid Hydrochlorid (PHMB) | |

Es hat sich überraschend gezeigt, dass der Einsatz aliphatischer Diole, wie beispielsweise das in den Beispielen 3 und 4 eingesetzte 1,2-Octandiol eine Reduzierung des einzusetzenden antimikrobiellen Wirkstoffs bei gleichem antimikrobiellem Effekt ermöglicht. Dies verringert weiterhin die Zytotoxizität, da für den gleichen antimikrobiellen Effekt eine niedrigere Konzentration des antimikrobiellen Wirkstoffs benötigt wird.

## Patentansprüche

1. Antimikrobielle Öl in Wasser Emulsion, umfassend
a) 0,005 bis 0,2 Gew.-% mindestens einer aliphatischen Komponente, die eine oder mehrere Guanid- und/oder Biguanid-Gruppe(n) aufweist,
b) mindestens ein Phospholipid und
c) mindestens eine Ölkomponente, wobei das Gewichtsverhältnis von Komponente a) zu Komponente b) 1:4 bis 1:40 ist.

2. Emulsion gemäß Anspruch 1, **dadurch gekennzeichnet, dass** die Ölkomponente ein mittelkettiges Triglycerid (MCT) umfasst, wobei das mittelkettige Triglycerid Glycerin ist, welches mit Fettsäuren verestert ist, die zumindest 90 % aus Caprylsäure (C₈) und Caprinsäure (C₁₀) bestehen und, dass die Komponente c) mindestens ein Pflanzenöl umfasst.

3. Emulsion gemäß Anspruch 2, **dadurch gekennzeichnet, dass** das Gewichtsverhältnis von mittelkettigem Triglycerid zu Pflanzenöl 1:10 bis 10:1 beträgt.

4. Emulsion gemäß mindestens einem der Ansprüche 1 bis 3, **dadurch gekennzeichnet, dass** die Komponente a) Polyhexamethylenbiguanid oder Polyhexamethylenbiguanidiniumhydrochlorid oder Mischungen aus Polyhexamethylenbiguanid und Polyhexamethylenbiguanidiniumhydrochlorid enthält.

5. Emulsion gemäß mindestens einem der Ansprüche 1 bis 3, **dadurch gekennzeichnet, dass** die Komponente a) erhältlich ist durch Polykondensation eines Guanidinsäureadditionssalzes mit einem Amingemisch, welches wenigstens ein Diamin und/oder ein Triamin enthält, wobei vorzugsweise wenigstens ein Amin ausgewählt ist aus der Gruppe, die aus
i) Diamin, das wenigstens einen cycloaliphatischen Rest aufweist, und
ii) Dialkylentriamin besteht.

6. Emulsion gemäß mindestens einem der Ansprüche 1 bis 5, **dadurch gekennzeichnet, dass** das Gewichtsverhältnis von Komponente a) zu Komponente b) 1:6 bis 1:20 ist.

7. Emulsion gemäß mindestens einem der Ansprüche 1 bis 6, **dadurch gekennzeichnet, dass** die Emulsion zusätzlich ein oder mehrere Alkandiol(e) aufweist.

8. Pharmazeutische Zubereitung umfassend eine Emulsion gemäß mindestens einem der Ansprüche 1 bis 7.

9. Pharmazeutische Zubereitung gemäß Anspruch 8 zur Verwendung in der Prophylaxe und Therapie von Infektionen, insbesondere von Infektionen an empfindlichen Körperregionen, beispielsweise dem Peritoneum, der Bauchhöhle, der Harnblase, des Harnleiters, des zentralen Nervensystems und hyalinem Gelenkknorpeln.

10. Pharmazeutische Zubereitung gemäß Anspruch 8 zur Verwendung in der Prophylaxe und Therapie atopischer Dermatiden, infizierter Ekzeme oder Dermatomykosen.

11. Antiseptikum umfassend eine Emulsion gemäß mindestens einem der Ansprüche 1 bis 7.

12. Antiseptikum gemäß Anspruch 11, zur Verwendung in der Prophylaxe und Behandlung von Infektionen der Haut, der Schleimhaut, des Genitalbereichs, der Augen, der Nasenhöhlen, insbesondere bei MRSA-Besiedlung, der Mundhöhle sowie von Wunden.

13. Verwendung der Emulsion gemäß mindestens einem der Ansprüche 1 bis 7 zur Herstellung einer festen oder gelartigen Wundauflage.

14. Wundauflage umfassend eine antimikrobielle Öl in Wasser Emulsion gemäß mindestens einem der Ansprüche 1 bis 7.

15. Verfahren zur Herstellung einer Emulsion gemäß mindestens einem der Ansprüche 1 bis 7 umfassend die Schritte:
α) Herstellung einer Öl in Wasser Emulsion umfassend
b) mindestens ein Phospholipid und
c) mindestens einer Ölkomponente und
β) Vermischen der in Schritt α) hergestellten Emulsion mit einer wässrigen Lösung umfassend
a) mindestens eine aliphatische Komponente, die eine oder mehrere Guanid- und/oder Biguanid-Gruppe(n) aufweist,
wobei das Gewichtsverhältnis von Komponente a) zu Phospholipid 1:4 bis 1:40 beträgt und die Komponente a) in der Emulsion in einer Menge von 0,005 bis 0,2 Gew.-%, bezogen auf das Gesamtgewicht der Emulsion, vorliegt.

## Claims

1. An antimicrobial oil-in-water emulsion, comprising:
a) from 0.005% to 0.2% by weight of at least one aliphatic component having one or more guanide and/or biguanide group(s);
b) at least one phospholipid; and
c) at least one oil component, wherein the weight ratio of component a) to component b) is from 1:4 to 1:40.

2. The emulsion according to claim 1, **characterized in that** said oil component comprises a medium-chain triglyceride (MCT), wherein said medium-chain triglyceride is glycerol esterified with fatty acids consisting of caprylic acid (C₈) and capric acid (C₁₀) to at least 90%, and that component c) comprises at least one vegetable oil.

3. The emulsion according to claim 2, **characterized in that** the weight ratio of medium-chain triglyceride to vegetable oil is from 1:10 to 10:1.

4. The emulsion according to at least one of claims 1 to 3, **characterized in that** component a) contains polyhexamethylenebiguanide, or polyhexamethylenebiguanidinium hydrochloride, or mixtures of polyhexamethylenebiguanide and polyhexamethylenebiguanidinium hydrochloride.

5. The emulsion according to at least one of claims 1 to 3, **characterized in that** component a) is obtainable by polycondensation of a guanidine acid addition salt with a mixture of amines containing at least one diamine and/or triamine, wherein preferably at least one amine is selected from the group consisting of:
i) diamine having at least one cycloaliphatic radical, and
ii) dialkylene triamine.

6. The emulsion according to at least one of claims 1 to 5, **characterized in that** the weight ratio of component a) to component b) is from 1:6 to 1:20.

7. The emulsion according to at least one of claims 1 to 6, **characterized in that** said emulsion additionally contains one or more alkanediol(s).

8. A pharmaceutical formulation comprising an emulsion according to at least one of claims 1 to 7.

9. The pharmaceutical formulation according to claim 8 for use in the prophylaxis and therapy of infections, especially infections in sensitive body zones, for example, the peritoneum, the abdominal cavity, the urinary bladder, the ureter, the central nervous system, and hyaline joint cartilage.

10. The pharmaceutical formulation according to claim 8 for use in the prophylaxis and therapy of atopic dermatides, infected ekzemas, or dermatomycoses.

11. An antiseptic comprising an emulsion according to at least one of claims 1 to 7.

12. The antiseptic according to claim 11 for use in the prophylaxis and treatment of infections of the skin, the mucosa, the genital area, the eyes, the nasal cavity, especially when colonized with MRSA, the oral cavity, and of wounds.

13. Use of the emulsion according to at least one of claims 1 to 7 for preparing a solid or gel-like wound dressing.

14. A wound dressing comprising an antimicrobial oil-in-water emulsion according to at least one of claims 1 to 7.

15. A process for preparing an emulsion according to at least one of claims 1 to 7, comprising the steps of :
α) preparing an oil-in-water emulsion comprising
b) at lest one phospholipid, and
c) at least one oil component; and
β) mixing the emulsion prepared in step α) with an aqueous solution comprising
a) at least one aliphatic component having one or more guanide and/or biguanide group(s);
wherein the weight ratio of component a) to the phospholipid is from 1:4 to 1:40, and component a) is present in the emulsion in an amount of from 0.005% to 0.2% by weight, based on the total weight of the emulsion.

## Revendications

1. Emulsion huile dans l'eau antimicrobienne, comprenant
a) 0,005 à 0,2 % en poids d'au moins un composant aliphatique contenant un ou plusieurs groupes guanide et/ou biguanide,
b) au moins un phospholipide, et
c) au moins un composant huileux, dans laquelle le rapport pondéral de composant a) à composant b) est de 1:4 à 1:40.

2. Emulsion selon la revendication 1, **caractérisée en ce que** ledit composant huileux comprend un triglycéride à chaîne moyenne (TCM), dans laquelle le triglycéride à chaîne moyenne est le glycérol estérifié par des acides gras consistant en au moins 90% d'acide caprylique (C₈) et d'acide caprique (C₁₀), et que le composant c) comprend au moins une huile végétale.

3. Emulsion selon la revendication 2, **caractérisée en ce que** le rapport pondéral du triglycéride à chaîne moyenne à l'huile végétale est de 1:10 à 10:1.

4. Emulsion selon l'une au moins des revendications 1 à 3, **caractérisée en ce que** le composant a) contient du polyhexaméthylène biguanide, ou du chlorhydrate de polyhexaméthylène biguanide, ou des mélanges de polyhexaméthylène biguanide est de chlorhydrate de polyhexaméthylène biguanide.

5. Emulsion selon l'une au moins des revendications 1 à 3, **caractérisée en ce que** le composant a) peut être obtenu par la polycondensation d'un sel d'addition d'acide de guanidine avec un mélange d'amines contenant au moins une diamine et/ou une triamine, de préférence dans lequel au moins une amine est choisie dans le groupe consistant en
i) diamine contenant au moins un radical cycloaliphatique, et
ii) triamine de dialkylène.

6. Emulsion selon l'une au moins des revendications 1 à 5, **caractérisée en ce que** le rapport pondéral de composant a) à composant b) est de 1:6 à 1:20.

7. Emulsion selon l'une au moins des revendications 1 à 6, **caractérisée en ce que** l'émulsion contient en outre un ou plusieurs alcanediols.

8. Formulation pharmaceutique comprenant une émulsion selon l'une au moins des revendications 1 à 7.

9. Formulation pharmaceutique selon la revendication 8 destinée à être utilisée dans la prophylaxie et la thérapie d'infections, notamment des infections dans les zones du corps sensibles, par exemple le péritoine, la cavité abdominale, la vessie, l'uretère, le système nerveux central et le cartilage hyalin articulaire.

10. Formulation pharmaceutique selon la revendication 8 destinée à être utilisée dans la prophylaxie et la thérapie de dermatites atopiques, d'eczémas infectés, ou de dermatomycoses.

11. Antiseptique comprenant une émulsion selon l'une au moins des revendications 1 à 7.

12. Antiseptique selon la revendication 11 destiné à être utilisé dans la prophylaxie et le traitement d'infections de la peau, de la muqueuse, de la région génitale, des yeux, des cavités nasales, notamment dans un cas de colonisation SARM, de la cavité orale, et de blessures.

13. Utilisation de l'émulsion selon l'une au moins des revendications 1 à 7 pour la préparation d'un pansement solide ou de type gel.

14. Pansement comprenant une émulsion huile dans l'eau antimicrobienne selon l'une au moins des revendications 1 à 7.

15. Procédé pour la préparation d'une émulsion selon l'une au moins des revendications 1 à 7, comprenant les étapes consistant à:
α) préparer une émulsion huile dans l'eau comprenant
b) au moins un phospholipide, et
c) au moins un composant huileux, et
β) mélanger l'émulsion préparée dans l'étape α) avec une solution aqueuse comprenant
a) au moins un composant aliphatique contenant un ou plusieurs groupes guanide et/ou biguanide,
dans lequel le rapport pondéral de composant a) à composant b) est de 1:4 à 1:40, et le composant a) est présent dans l'émulsion en une quantité de 0,005 à 0,2 % en poids, par rapport au poids total de l'émulsion.
